# EUROPEAN PATENT SPECIFICATION

(11) **EP 3 845 900 B1**
(45) Date of publication and mention of the grant of the patent: **23.04.2025**
(21) Application number: 19853478.6
(22) Date of filing: 28.08.2019
(51) Int. Cl.: G01N 33/543

(54) **CHROMATOGRAPHY KIT, AND CHROMATOGRAPHY METHOD**
CHROMATOGRAFIEKIT UND CHROMATOGRAFIEVERFAHREN
KIT DE CHROMATOGRAPHIE ET MÉTHODE DE CHROMATOGRAPHIE

(30) Priority: 29.08.2018 JP 2018160179
(43) Date of publication of application: 07.07.2021
(73) Proprietor: FUJIFILM Corporation, Tokyo 106-8620 (JP)
(72) Inventor: OYABU, Natsuki, Ashigarakami-gun, Kanagawa 258-8538 (JP); OHARA, Tomoya, Ashigarakami-gun, Kanagawa 258-8538 (JP); KATADA, Junichi, Ashigarakami-gun, Kanagawa 258-8538 (JP); WADA, Atsuhiko, Ashigarakami-gun, Kanagawa 258-8538 (JP)
(74) Representative: Dehns Germany Partnerschaft mbB
(86) International application number: PCT/JP2019/033775
(87) International publication number: WO 2020/045524

(56) References cited:
- EP-A1- 2 602 619
- EP-A1- 3 246 704
- EP-A2- 2 506 013
- WO-A1-2017/104143
- WO-A1-2017/104143
- JP-A- 2002 296 278
- JP-A- 2012 037 253
- JP-A- 2012 211 849
- JP-A- 2013 181 935
- JP-A- 2013 181 935
- US-A1- 2018 120 250

## Description

### Field of the Invention

The present invention relates to a chromatographic kit including a label substance, a nonionic surfactant, a Good's buffer, casein, a porous carrier, a compound containing silver, and a reducing agent capable of reducing silver ions. The present invention further relates to a chromatographic method in which a label substance, a nonionic surfactant, a Good's buffer, casein, a porous carrier, a compound containing silver, and a reducing agent capable of reducing silver ions are used. The Good's buffer included in the chromatographic kit and used in the chromatographic method according to the invention is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES.

### Description of the Related Art

Among immunoassay methods, a chromatographic method is generally utilized, because operation is easy and measurement can be performed within a short period of time. Competitive responses or sandwich-based responses are widely used as an immune response used in the chromatographic method. Among them, sandwich-based responses are the mainstream in the chromatographic method, and in a typical example thereof, the following operation is performed in order to detect a test substance composed of antigens in a specimen. First, fine particles sensitized with an antibody against an antigen which is a test substance are immobilized as solid-phase fine particles on a chromatographic carrier, or an antibody itself is directly immobilized on a chromatographic carrier, and thereby a chromatographic carrier having a reaction site is prepared. Meanwhile, labeled fine particles are sensitized with an antibody against a test substance to prepare sensitized labeled fine particles. The sensitized labeled fine particles are then chromatographically moved together with a specimen on a chromatographic carrier. By the above operation, the immobilized antibody serves as an immobilization reagent at a reaction site formed on the chromatographic carrier, and the sensitized labeled fine particles specifically bind to this immobilized antibody via an antigen which is a test substance. As a result, by visually determining the presence or absence or a degree of signals generated by the sensitized labeled fine particles trapped at the reaction site, it is possible to measure the presence or absence or an amount of a test substance in a specimen.

Patent Document 1 discloses use of a reagent composition for an immunochromatographic method, which contains a nonionic surfactant, a N,N-bis(2-hydroxyethyl)glycine buffer, and casein, as a test sample treatment liquid or spread liquid in a case of detecting a detection target in a test sample by an immunochromatographic method.

Patent Document 2 discloses a pretreatment method including a step of bringing a non-specific reaction inhibitor containing a protease into contact with a urine specimen in an immunochromatographic method for detecting or quantitatively determining a polysaccharide antigen in a urine sample, and discloses that a buffer solution such as a phosphate buffer solution, a Tris buffer solution, or a Good's buffer solution, a protein (such as bovine serum albumin, casein, or gelatin), a nonionic surfactant, and the like may be added as a solution for pretreatment and a spread liquid.

### Prior Art Documents

### Patent Documents

Patent Document 1: JP2012-037253A
Patent Document 2: JP2018-036119A

### SUMMARY OF THE INVENTION

As a chromatographic method, a method of spreading a specimen liquid, from which an antigen in a test sample has been extracted, on a test strip is generally used. However, in a case of test samples with a wide range of pH such as urine test samples, defects may occur, in which a pH cannot be adjusted with existing specimen liquids, foreign substances having an isoelectric point in a test sample are non-specifically adsorbed on a test strip, and thereby false positives are generated. In particular, in a chromatographic method in which signal amplification is performed in order to avoid a problem of false negatives indicating that an antigen is not detected due to a low sensitivity, a test line stands out as compared to a chromatographic method in which amplification is not performed, and therefore defects, which are generation of false positives, occur in many cases.

Due to the above-mentioned problems, it is common in a chromatographic kit to dilute a test sample for the purpose of adjusting a pH of the test sample. However, in a silver amplification chromatographic method having high-sensitivity, false positives are conspicuous in a case of merely using general pH buffers, and a predetermined antigen cannot be qualitatively and quantitatively determinined in many cases.

An object of the present invention is to provide a chromatographic kit and a chromatographic method which have high-sensitivity and in which occurrence of false positives is inhibited.

As a result of intensive studies to achieve the above-mentioned object, the inventors of the present invention have found that measurement can be performed with high-sensitivity while still inhibiting generation of false positives by spreading a test substance using a spread liquid containing a nonionic surfactant, a Good's buffer as specified in the claims, and casein, and amplifying signals using a compound containing silver and a reducing agent capable of reducing silver ions. Therefore, they have completed the present invention.

That is, according to the present invention, the following inventions are provided.
[1] A chromatographic kit comprising: a label substance modified with a first binding substance for a test substance; a nonionic surfactant; a Good's buffer, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES; casein; a porous carrier including a second binding substance for the test substance or a binding substance for the first binding substance; a compound containing silver; and a reducing agent reducing silver ions.
[2] The chromatographic kit according to [1], in which the nonionic surfactant is a polysorbate surfactant.
[3] The chromatographic kit according to [1] or [2], in which the label substance is a metal particle.
[4] The chromatographic kit according to any one of [1] to [3], in which the porous carrier includes
   a label substance-holding region having the label substance modified with the first binding substance for the test substance,
   a label substance-trapping region having the second binding substance for the test substance or the binding substance for the first binding substance, and
   a coloring region having a coloring reagent for detecting the reducing agent reducing silver ions.
[5] The chromatographic kit according to any one of [1] to [4], in which the porous carrier is a nitrocellulose carrier.
[6] The chromatographic kit according to any one of [1] to [5], in which the first binding substance and the second binding substance are antibodies.
[7] A chromatographic method comprising:
   a spreading step of spreading a spread liquid onto a porous carrier including a label substance-trapping region having a second binding substance for the test substance or a binding substance for the first binding substance, the spread liquid containing a specimen containing a test substance, a label substance modified with a first binding substance for the test substance, a nonionic surfactant, a Good's buffer, and casein, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES;
   a step of trapping a complex body of the test substance and the label substance modified with the first binding substance for the test substance in a label substance-trapping region; and
   a step of amplifying a signal of the label substance of the trapped complex body using a compound containing silver and a reducing agent reducing silver ions.
[8] The chromatographic method according to [7], in which a final concentration of the nonionic surfactant in the spread liquid is 0.05 to 2.5 mass%.
[9] The chromatographic method according to [7] or [8], in which the nonionic surfactant is a polysorbate surfactant.
[10] The chromatographic method according to any one of [7] to [9], in which the label substance is a metal particle.
[11] The chromatographic method according to any one of [7] to [10], the method further comprising detecting a label substance having an average particle size of equal to or more than 1 µm and equal to or less than 20 µm in a case of detection.
[12] The chromatographic method according to any one of [7] to [11], in which the porous carrier is a nitrocellulose carrier.
[13] The chromatographic method according to any one of [7] to [12], in which the specimen containing the test substance is nasal discharge, a nasal swab, a pharyngeal swab, a nasal aspirate, or urine.

According to a chromatographic kit and a chromatographic method of the aspects of the present invention, measurement can be performed with high-sensitivity while still inhibiting generation of false positives.

### BRIEF DESCRIPTION OF THE DRAWINGS

Fig. 1 is a perspective view showing an example of a chromatographic kit.
Fig. 2 is an exploded schematic perspective view showing the example of the chromatographic kit.
Fig. 3 is a schematic side view showing a positional relationship between an inspection strip, and a first pot and a second pot.

### DESCRIPTION OF THE PREFERRED EMBODIMENTS

Hereinafter, embodiments of the present invention will be described in detail.

Numerical value ranges expressed using "to" in the present specification mean a range including numerical values described before and after "to" as a minimum value and a maximum value.

A chromatographic kit of the embodiment of the present invention is a chromatographic kit including: a label substance modified with a first binding substance for a test substance; a nonionic surfactant; a Good's buffer, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES; casein; a porous carrier including a second binding substance for the test substance or a binding substance for the first binding substance; a compound containing silver; and a reducing agent reducing silver ions.

A chromatographic method of the embodiment of the present invention is a method including:
a spreading step of spreading a spread liquid onto a porous carrier including a second binding substance for the test substance or a binding substance for the first binding substance, the spread liquid containing a specimen containing a test substance, a label substance modified with a first binding substance for the test substance, a nonionic surfactant, a Good's buffer, and casein, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES;
a step of trapping a complex body of the test substance and the label substance modified with the first binding substance for the test substance in the label substance-trapping region; and
a step of amplifying a signal of the label substance of the trapped complex body using a compound containing silver and a reducing agent reducing silver ions.

According to the chromatographic kit and chromatographic method of the embodiment of the present invention, qualitative or quantitative analysis of a test substance can be performed.

### 1. Chromatography

In general, a chromatographic method is a technique of simply, rapidly, and specifically determining and measuring a test substance by the following technique. That is, a membrane (porous carrier) is used as an immobilized phase, where the membrane may have a label substance-trapping region having at least one detection site having a binding substance (specifically, an antibody and the like) capable of binding to a test substance. On this porous carrier, a liquid containing a label substance modified with a first binding substance against a test substance is moved chromatographically as a moving layer to reach the label substance-trapping region having the detection site while the test substance and the label substance specifically bind to each other. The technique is a technique of qualitatively and quantitatively analyzing the presence of a test substance in a test specimen visually or using an appropriate device by utilizing that, in the detection site of the label substance-trapping region, a complex body of the test substance and the label substance specifically binds to an immobilized second binding substance, and thereby the label substance is concentrated in the second binding substance only in a case in which the test substance is present in a test specimen.

In a chromatographic method of the embodiment of the present invention, using two kinds of amplification reagents used for amplifying signals of a label substance, specifically, a compound containing silver and a reducing agent capable of reducing silver ions, it is possible to amplify signals by an amplification reaction using, as a nucleus, a complex body of a label substance and a test substance bound to an immobilization reagent on a label substance-trapping region, and as a result, it is possible to achieve high-sensitivity. According to the present invention, rapid chromatography with high-sensitivity can be performed.

### 2. Specimen containing test substance (also called test specimen)

A test specimen that can be analyzed using the chromatographic method and the kit of the embodiment of the present invention is not particularly limited as long as it is a specimen that may contain a test substance. Examples thereof include biological specimens, particularly body fluids (for example, blood, serum, plasma, spinal fluid, tears, sweat, urine, pus, nasal discharge, nasal swab, pharyngeal swab, nasal aspirate, or sputum) of animals (particularly humans), or excrements (for example, feces), organs, tissues, mucous membranes and skin, scraped test sample (swabs) that contain these substances, or animals and plants themselves or dried substances thereof. The specimen containing the test substance is preferably nasal discharge, a nasal swab, a pharyngeal swab, a nasal aspirate, or urine.

It is possible to use the test specimen as it is, or in a form of an extraction liquid obtained by extracting the test specimen using an appropriate solvent for extraction, in a form of a diluent solution obtained by diluting an extraction liquid with an appropriate diluent, or in a form of in which an extraction liquid has been concentrated by an appropriate method. As the solvent for extraction used in the present invention, it is possible to use a solvent used in a general immunological analysis method (for example, water, physiological saline, a buffer solution, and the like), or a water-miscible organic solvent that enables performing of a direct antigen-antibody reaction by diluting with such a solvent.

### 3. Spread liquid

A spread liquid used for spreading the specimen containing the test substance on a porous carrier contains a nonionic surfactant, a Good's buffer, and casein.

Examples of nonionic surfactants include polyoxyethylene alkyl ether, polyoxyethylene/polyoxypropylene alkyl ether, polyoxyethylene sorbitan fatty acid ester (Tween (registered trademark) series), polyoxyethylene p-t-octylphenyl ether (Triton (registered trademark) series), polyoxyethylene p-t-nonylphenyl ether (Triton (registered trademark) N series), alkyl polyglucoside, fatty acid diethanolamide, alkyl monoglyceryl ether, and the like. Among them, polyoxyethylene sorbitan fatty acid ester (Tween (registered trademark) series), which is a polysorbate surfactant, is preferable, and, for example, Tween (registered trademark) 40, Tween (registered trademark) 60, and Tween (registered trademark) 80 are particularly preferable.

A content of the nonionic surfactant in the spread liquid is preferably within a range of 0.01 to 10 mass%, more preferably within a range of 0.05 to 5 mass%, and even more preferably within a range of 0.1 to 1 mass%.

Good's buffers refer to buffers originally described by Norman Good et al. in 1966 (N.E. Good et al. (1966). Hydrogen Ion Buffers for Biological Research. Biochemistry 5(2):467-477).

Specific examples of Good's buffers are shown in Table 1. In the present invention, it is preferable to use a Good's buffer having a buffer capacity of pH 8 to pH 9 (for example, pH 8.5).

**[Table 1]**

| Shorthand | Name |
|---|---|
| ACES | N-(2-Acetamido)-2-aminoethane sulfonic acid |
| ADA | N-(2-Acetamido)iminodiacetic acid |
| BES | N,N-Bis(2-hydroxyethyl)-2-aminoethanesulfonic acid |
| Bicine | N,N-Bis(2-hydroxyethyl)glycine |
| Bis-Tris | Bis(2-hydroxyethyl)iminotris(hydroxymethyl)methane |
| CAPS | N-Cyclohexyl-3-aminopropanesulfonic acid |
| CAPSO | N-Cyclohexyl-2-hydroxy-3-aminopropanesulfonic acid |
| CHES | N-Cyclohexyl-2-aminoethanesulfonic acid |
| DIPSO | 3-[N,N-Bis(2-hydroxyethyl)amino]-2-hydroxypropanesulfonic acid |
| EPPS | 3-[4-(2-Hydroxyethyl)-1-piperazinyl]propanesulfonic acid |
| HEPES | 3-[4-(2-Hydroxyethyl)-1-piperazinyl]ethanesulfonic acid |
| HEPES-Na | 2-[4-(2-Hydroxyethyl)-1-piperazinyl]ethanesulfonic acid sodium salt |
| HEPPSO | 2-Hydroxy-3-[4-(2-hydroxyethyl)-1-piperazinyl]propanesulfonic acid monohydrate |
| MES | 2-Morpholinoethanesulfonic acid monohydrate |
| MOPS | 3-Morpholinopropanesulfonic acid |
| MOPSO | 2-Hydroxy-3-morpholinopropanesulfonic acid |
| PIPES | Piperazine-1,4-bis(2-ethanesulfonic acid) |
| POPSO | Piperazine-1,4-bis(2-hydroxy-3-propanesulfonic acid) |
| TAPS | N-Tris(hydroxymethyl)methyl-3-aminopropanesulfonic acid |
| TAPSO | 2-Hydroxy-N-tris(hydroxymethyl)methyl-3-aminopropanesulfonic acid |
| TES | N-Tris(hydroxymethyl)methyl-2-aminoethanesulfonic acid |
| Tricine | N-[Tris(hydroxymethyl)methyl]glycine |

Tricine, HEPPSO, TAPS or CHES are used in the present invention.

A concentration of the Good's buffer in the spread liquid is preferably within a range of 10 to 1,000 mmol/L, more preferably within a range of 50 to 500 mmol/L, and even more preferably within a range of 100 to 300 mmol/L.

Casein used in the present invention is not particularly limited. Casein can be obtained from milk according to a conventional method. For example, casein can be separated from milk by precipitation in a pH environment below pH 4.6, which is an isoelectric point of casein. Casein may be casein separated according to a conventional method, or a commercially available product may be used. In addition, as casein, casein in which a molecular weight has been lowered by physical decomposition such as heating or pressurization, chemical decomposition with acid/alkali or the like, or enzymatic decomposition with proteolytic enzyme may be used.

A content of the casein in the spread liquid is preferably within a range of 0.001 to 1 mass%, more preferably within a range of 0.005 to 0.5 mass%, and even more preferably within a range of 0.01 to 0.1 mass%.

### 4. Constitution

In the chromatographic kit of the embodiment of the present invention, a chromatographic strip can be incorporated and used. The chromatographic strip that can be used is not particularly limited as long as it is a chromatographic strip that can be used in general chromatographic methods.

The chromatographic strip that can be used in the present invention has a label substance-holding region and a label substance-trapping region from the upstream direction to the downstream direction of a spreading direction of a test specimen containing a test substance. In a preferred aspect, the chromatographic strip further has a region (coloring region) containing a coloring reagent. A more preferred aspect of the present invention is an aspect in which the region containing a coloring reagent is located in the downstream direction of the label substance-trapping region. Furthermore, an aspect, in which a specimen-added pad, a label substance-holding pad having a label substance-holding region (for example, gold colloid antibody-holding pad), an antibody-immobilized membrane that is a porous carrier (for example, an antibody-immobilized membrane having a label substance-trapping region), and a water absorption pad are disposed in this order on a pressure-sensitive adhesion sheet, is preferably used. The antibody-immobilized membrane that is a porous carrier has a label substance-trapping region that is a region having at least one detection site on which an antibody that specifically binds to a test substance is immobilized, or as desired, the antibody-immobilized membrane may further have a control site (sometimes referred to as a control region) which is a region in which an antibody for control or an antigen is immobilized.

The label substance-holding pad which has the label substance-holding region and can be used in the present invention can be prepared by preparing a suspension containing the label substance, applying the suspension to an appropriate water absorption pad (for example, glass fiber pad), and then drying the suspension.

### 4-1. Label substance

As the label substance used in the present invention, it is preferable to use a label substance containing a metal as a label used for labeling the first binding substance against a test substance. The label substance is more preferably a metal particle. As the type of metal that can be used in the present invention, noble metals such as gold, silver, and platinum, iron, lead, copper, cadmium, bismuth, antimony, tin, or mercury can be preferably used, or compounds thereof can be used. Noble metals such as gold, silver, and platinum can be more preferably used. As a preferred form of the label substance containing a metal which can be used in the present invention, it is possible to use a metal colloid label or a metal sulfide label. In the present invention, as the metal colloid label, platinum colloid, gold colloid, silver colloid, iron colloid, aluminum hydroxide colloid, or the like can be preferably used, and as the metal sulfide label, each of sulfides of iron, silver, lead, copper, cadmium, bismuth, antimony, tin, or mercury can be preferably used. In the present invention, platinum colloid, gold colloid, or silver colloid can be more preferably used, and gold colloid can be most preferably used. In a case where gold colloid particles are used as the metal colloid label, commercially available gold colloid particles may be used. Alternatively, gold colloid particles can be prepared by a conventional method such as a method of reducing chlorauric acid with sodium citrate (Nature Physical Science, 241 (1973) 20, and the like).

An average particle size of the metal colloid is preferably about 1 nm to 500 nm, more preferably 3 to 100 nm, and particularly preferably 5 to 60 nm. An average particle size of the metal colloid used in the present invention can be measured with a commercially available particle size distribution meter or the like. As a method of measuring particle size distribution, optical microscopy, confocal laser microscopy, electron microscopy, atomic force microscopy, a static light scattering method, a laser diffraction method, a dynamic light scattering method, a centrifugal sedimentation method, an electric pulse measurement method, a chromatographic method, an ultrasonic attenuation method, and the like are known, and devices corresponding to the respective principles are commercially available.

As a method of measuring an average particle size, a dynamic light scattering method can be preferably used because of a particle size range and ease of measurement. Examples of commercially available measuring devices using dynamic light scattering include NANOTRAC UPA (Nikkiso Co., Ltd.), a dynamic light scattering type particle size distribution measuring device LB-550 (HORIBA, Ltd.), a concentrated particle size analyzer FPAR-1000 (Otsuka Electronics Co., Ltd.), and the like. In the present invention, an average particle size is obtained as a value of a median diameter (d = 50) measured at a measurement temperature of 25°C.

According to the present invention, in chromatography using a metal colloid label or metal sulfide label, another metal alloy label (hereinafter sometimes referred to as a metallic label), or a metal-containing polymer particle label as a label substance for detection, signals of the metallic label can be amplified. Specifically, in a case where, after formation of a complex body of a test substance and a label for detection, silver ions supplied from a compound containing silver such as an inorganic silver salt or an organic silver salt are brought into contact with a reducing agent capable of reducing silver ions, the silver ions are reduced by the reducing agent, and thereby silver particles are generated, the silver particles are deposited on a metallic label with the metallic label as a nucleus, and therefore, the metallic label is amplified, and analysis of the test substance can be performed with high-sensitivity. That is, in the chromatographic method of the embodiment of the present invention, the silver particles generated by the reducing action of silver ions by the reducing agent are used to perform a reaction for depositing on a label of an immune complex body, and signals thus amplified are analyzed.

### 4-2. Binding substance

The label substance is modified with the first binding substance for a test substance in the present invention. The first binding substance may be any binding substance as long as it is a compound, which has an affinity for a test substance, such as an antibody against a test substance (antigen), an antigen against a test substance (antibody), an aptamer against a test substance (protein, low-molecular compound, and the like).

The chromatographic kit of the embodiment of the present invention includes the second binding substance for the test substance or the binding substance for the first binding substance in label substance-trapping region. The second binding substance for a test substance may be any binding substance as long as it is a compound, which has an affinity for a test substance, such as an antibody against a test substance (antigen), an antigen against a test substance (antibody), an aptamer against a test substance (protein, low-molecular compound, and the like). Furthermore, the second binding substance and the first binding substance may be different from each other or may be the same as each other. The binding substance for the first binding substance may be a test substance itself or may be a compound having a site recognized by the first binding substance. Examples thereof include a compound obtained by binding of a derivative of a test substance and a protein (for example, BSA or the like), and the like.

It is preferable that the first binding substance be an antibody and/or the second binding substance be an antibody.

In the chromatographic method of the embodiment of the present invention, an antibody having specificity against a test substance is not particularly limited. For example, it is possible to use antisera prepared from animal sera immunized with the test substance, immunoglobulin fractions purified from antiserum, monoclonal antibodies obtained by cell fusion using animal spleen cells immunized with the test substance, or fragments thereof [for example, F(ab')₂, Fab, Fab', or Fv]. Preparation of these antibodies can be performed by a conventional method.

In the present invention, a method of modifying the label substance using the first binding substance can be performed according to, for example, a conventionally known method described below (for example, The Journal of Histochemistry and Cytochemistry, 30, 7 (1982) 691-696) in a case of binding a metal colloid and a specific binding substance to each other. As a specific example, a metal colloid and a specific binding substance (for example, an antibody) are mixed in an appropriate buffer solution at room temperature for 5 minutes or longer. After the reaction, a precipitate obtained by centrifugation is dispersed in a solution containing a dispersant such as polyethylene glycol, and thereby a desired specific binding substance labeled with the metal colloid can be obtained.

### 4-3. Porous carrier

As the porous carrier that can be used in the present invention, a nitrocellulose carrier (such as a nitrocellulose membrane), a cellulose membrane, an acetyl cellulose membrane, a polysulfone membrane, a polyether sulfone membrane, a nylon membrane, a glass fiber, a non-woven fabric, a cloth, a thread, and the like are particularly preferable.

In the present invention, a detection site, on which the second binding substance for the test substance or the binding substance for the first binding substance is immobilized, is provided on the label substance-trapping region of the porous carrier. The second binding substance for the test substance or the binding substance for the first binding substance may be directly immobilized on a part of the porous carrier by a physical or chemical bond, and thereby the detection site is formed, or the second binding substance for the test substance or the binding substance for the first binding substance may physically or chemically bind to fine particles such as latex particles, these fine particles are trapped on a part of the porous carrier and immobilized, and thereby the detection site is formed. The porous carrier is preferably used after immobilizing the second binding substance for the test substance or the binding substance for the first binding substance thereon and then subjecting the porous carrier to a non-specific adsorption prevention treatment such as a treatment with an inactive protein. The porous carrier of the present invention can also be preferably used in a form of having a plurality of binding sites, and as desired, it may further have the above-mentioned control site as a part of the label substance-trapping region.

### 4-4. Label substance-holding pad

In the present invention, an aspect in which a label substance-holding pad having a label substance-holding region, preferably a gold colloid-holding pad, is incorporated into the chromatographic kit and used is preferable. As a material of the label substance-holding pad, for example, cellulose filter paper, glass fiber, non-woven fabric, and the like can be preferably used, and it is possible to obtain the label substance-holding region by impregnating a certain amount of the label substance prepared as described above and drying it.

### 4-5. Specimen-added pad

It is preferable to use the chromatographic kit of the embodiment of the present invention by further incorporating a specimen-added pad thereto. For the specimen-added pad, an aspect in which the specimen-added pad has not only a function of receiving an added specimen containing a test substance but also has a function of filtering insoluble particles and the like in the specimen is preferable. Examples of materials of the specimen-added pad include materials having uniform characteristics such as cellulose filter paper, glass fibers, polyurethane, polyacetate, cellulose acetate, nylon, and cotton cloth. In addition, in order to prevent the test substance in the specimen from non-specifically adsorbing to a material of the specimen-added pad and lowering accuracy of analysis during analysis, a material forming the specimen-added pad can also be used after being treated to a non-specific adsorption prevention treatment in advance. In the present invention, the specimen-added pad may also serve as the label substance-holding pad having the label substance-holding region.

### 4-6. Water absorption pad

In the present invention, it is preferable to use the chromatographic kit by incorporating a water absorption pad thereto. The water absorption pad is a site that physically absorbs an added specimen by chromatographic migration and also absorbs and removes unreacted label substances that are not insolubilized in a detection part of a chromatographic carrier, and water absorbent materials such as cellulose filter paper, non-woven fabric, cloth, and cellulose acetate are used. Because a chromatographic speed after a chromatographic tip end portion of the added specimen reaches the water absorption pad depends on materials, sizes, and the like of the water absorption pad, it is possible to set a speed that is suitable for measurement of the test substance by selecting materials, sizes, and the like of the water absorption pad.

### 5. Coloring reagent for detecting reducing agent capable of reducing silver ions

In the chromatographic kit used in the present invention, a coloring reagent is preferably carried by a porous carrier.

In the present invention, it is preferable to use, for example, a compound that reacts with ions and develops color as the coloring reagent for detecting the reducing agent capable of reducing silver ions. Although a first amplification reagent will be described later in the present specification, a compound that reacts with Fe²⁺ ions and develops color can be used as a coloring reagent for the first amplification reagent in a case where the first amplification reagent is a reagent containing divalent iron ions (Fe²⁺), for example. As the compound that reacts with Fe²⁺ ions and develops color, it is possible to use a compound capable of developing color by forming a complex with Fe²⁺ ions. As specific examples of the compound that reacts with Fe²⁺ ions and develops color, it is possible to use compounds having a phenanthroline skeleton [for example, 1,10-phenanthroline, 5-methyl phenanthroline, 5-nitrophenanthroline, bathophenanthroline (4,7-diphenyl-1,10-phenanthroline), bathophenanthroline disulfate, and the like], or compounds having a bipyridine skeleton [for example, 2,2'-bipyridine and the like], and compounds having a phenanthroline skeleton can be preferably used. In addition, in a case in which a pH of an aqueous solution containing a test specimen and a pH of an aqueous solution containing the first amplification reagent are different from each other, it is possible to preferably use a reagent in which tint is changed because of structural change occurring due to H⁺ ions in order to detect the first amplification reagent. Particularly, in a case in which the aqueous solution containing the first amplification reagent is acidic (where a pH is lower than 7, and a concentration of H⁺ ions is high), as an pH indicator for an acidic region, it is preferable to appropriately select compounds and the like (for example, diazo-based coloring reagents such as methyl orange, methyl red, congo red, and methyl yellow, and sultone-based coloring reagents such as thymol blue, bromocresol green, bromocresol purple, and bromothymol blue), which react with H⁺ ions and develops color and which are well-known coloring reagents, in accordance with the pH of the aqueous solution containing the amplification reagent. Among them, 1,10-phenanthroline, bathophenanthroline, or bromocresol green can be more preferably used.

The coloring reagent is preferably a coloring reagent that does not substantially move in the porous carrier in a case where any of an aqueous solution containing a test specimen or an aqueous solution containing the reducing agent reducing silver ions is spread. Accordingly, LogP (partition coefficient in water and octanol) of the coloring reagent is preferably 4.0 or more, and more preferably 5.0 or more. An actual measurement value may be used as LogP, but a calculation value obtained from a chemical structure or the like can also be used as a simple judging method. As a method of calculating LogP, a calculation method used in ChemDraw Pro version 12 of Cambridge Soft is preferable. Responsiveness and LogP (according to ChemDraw Pro version 12) of representative coloring reagents are shown in Table 2.

**[Table 2]**

| Name of compound | Responsiveness | LogP |
|---|---|---|
| 2,2'-Bipyridine | Fe²⁺ response | 1.88 |
| Bathophenanthroline disulfonic acid | Fe²⁺ response | 0.52 |
| 1,10-Phenanthroline | Fe²⁺ response | 2.2 |
| 5-Methylphenanthroline | Fe²⁺ response | 2.69 |
| 5-Nitrophenanthroline | F^{e2} +response | 2.34 |
| Thymol blue | pH response | 4.01 |
| Methyl orange | pH response | 2.95 |
| Methyl red | pH response | 3.63 |
| Congo red | pH response | 3.63 |
| Methyl yellow | pH response | 4.76 |
| Bathophenanthroline | Fe²⁺ response | 5.55 |
| Bromocresol green | pH response | 7.99 |
| Bromocresol purple | pH response | 6.33 |
| Bromothymol blue | pH response | 8.8 |

A region having the coloring reagent is preferably located downstream of the label substance-trapping region having the detection site of the porous carrier. As a method of holding the coloring reagent in the chromatographic kit, there are a method of immersing a water absorption pad to be described later in a coloring reagent solution and drying under reduced pressure, a method of linearly applying in a downstream direction from a label substance-trapping region of a porous carrier, and the like.

In a case where the coloring reagent substantially moves in the porous carrier in a case where the aqueous solution containing the test specimen or the aqueous solution containing the first amplification reagent is spread, it is preferable that the coloring reagent be contained in the water absorption pad and used.

In a case where the coloring reagent does not substantially move in the porous carrier in a case where the aqueous solution containing the test specimen or the aqueous solution containing the first amplification reagent is spread, it is preferable to cause the porous carrier having the label substance-trapping region to carry the coloring reagent.

In the present invention, an aspect in which the coloring reagent is carried by the porous carrier is more preferable because then it is possible to display arrival of the first amplification reagent in the label substance-trapping region with a smaller time lag.

In the present invention, in a case where a region to which a test specimen containing a test substance is added, and a label substance-trapping region are provided in this order from an upstream direction to a downstream direction with respect to a spreading direction of the test specimen containing the test substance, an upstream direction and a downstream direction with respect to the spreading direction of the test specimen containing the test substance are defined in a case where the test specimen is spread by utilizing capillarity, suction power in a case where the water absorption pad is used, or the like. In a specific aspect of the present invention, in a case where a test specimen and the like are spread from a label substance-holding region toward a label substance-trapping region, a direction of the label substance-holding region is defined as an upstream direction, and a direction of the label substance-trapping region is defined as a downstream direction.

In a preferred aspect of the present invention, the first amplification reagent of the two kinds of amplification reagents used for amplifying signals of a label substance trapped in the label substance-trapping region is spread from the upstream direction of the label substance-trapping region to the downstream direction of the label substance-trapping region to detect physical or chemical changes in the region having the coloring reagent, and thereby it is possible to confirm that the label substance-trapping region is filled with the first amplification reagent. As the physical or chemical changes in the region having the coloring reagent, it is possible to detect changes in color development or fluorescence caused by a reaction between the first amplification reagent and the coloring reagent. Color development can be preferably detected. Such physical or chemical changes may be detected visually or may be detected using a detection device.

### 6. Method of inspection

A sandwich method and a competitive method which are specific embodiments for the chromatographic method of the embodiment of the present invention will be described below.

The sandwich method is not particularly limited, but for example, analysis of a test substance can be performed by the following procedure. First, a first binding substance for a test substance and a second binding substance for a test substance are prepared in advance. In addition, label substances are modified with the first binding substance in advance. In a case where the second binding substance is immobilized on an appropriate chromatographic carrier (porous carrier) (for example, nitrocellulose membrane, glass fiber membrane, nylon membrane, cellulose membrane, and the like) so as to serve as a label substance-trapping region, and this region is brought into contact with a test specimen (or an extraction liquid thereof) that may contain a test substance, binding to the second binding substance (for example, an antigen-antibody reaction with the second binding substance) occurs in a case where the test substance is present in the test specimen. In a case where an excess amount of label substances modified with the first binding substance is further brought into contact with the region at the same time of binding of the test substance and the second binding substance, or after binding thereof, a complex body consisting of the immobilized second binding substance, the test substance, and the label substances modified with the first binding substance is formed in a case where the test substance is present in the test specimen.

In the sandwich method, it is possible to determine the presence or absence of the test substance in the test specimen or measure an amount thereof by removing a label substance that did not form an immune complex body after the reaction of the immobilized second binding substance with the test substance, and the test substance with the first binding substance with which the label substances are modified is completed, and thereafter, by for example, observing a label substance-trapping region of a porous carrier as it is, and detecting or quantitatively determining a label substance. In the present invention, for example, a reducing agent and a silver ion-containing compound are supplied, and thereby signals from a label substance that has formed such a complex body are amplified and detected.

The competitive method is not particularly limited, but for example, analysis of a test substance can be performed by the following procedure. The competitive method is known as a technique of detecting an antigen of a low-molecular compound that cannot be assayed by the sandwich method. First, a first binding substance having specificity for a test substance (antigen) is prepared in advance. In addition, label substances are modified with the first binding substance in advance. The test substance itself, which is a binding substance for the first binding substance, or a compound which is similar to the test substance and has a site similar to that of the test substance, is immobilized on an appropriate binding substance-immobilized membrane (for example, nitrocellulose membrane, glass fiber membrane, nylon membrane, cellulose membrane, and the like) and used as a label substance-trapping region. In a case where a test specimen (or an extract thereof) that may contain the test substance (antigen), and a label substance modified with the first binding substance in advance are brought into contact with each other, and in a case where the test substance is not present in the test specimen, a complex body is formed from the first binding substance with which the label substance is modified, and the test substance itself, which is immobilized on the label substance-trapping region and has bonding properties with respect to the first binding substance, or the compound similar to the test substance. On the other hand, in a case where the test substance is present in the test specimen, because the test substance (antigen) binds to the first binding substance with which the label substance is modified, binding to the test substance itself, which immobilized on the label substance-trapping region and has bonding properties with respect to the first binding substance, or the compound similar to the test substance is hindered, and therefore a complex body is not formed.

After the reaction between the test substance itself or the compound similar to the test substance having a site similar to that of the test substance, and the first binding substance with which the label substance is modified is completed, the first binding substance with which an unbound label substance is modified is removed. Subsequently, an amount of the label substance modified with the first binding substance in the label substance-trapping region is detected or quantitatively determined, and thereby the presence/absence of the test substance in the test specimen can be determined, or an amount thereof can be measured. In the present invention, for example, a reducing agent capable of reducing silver ions and a compound containing silver are supplied, and thereby signals from a label substance that has formed a complex body are amplified and detected.

### 7. Amplification reagent

An amplification reagent is a reagent that can cause signal amplification by catalytically reacting due to an action of a label substance or a test substance and causing coloration of a compound, luminescence, and the like. The amplification reagent can used in a state of a solution containing a reagent, that is, an amplification liquid. Examples thereof include a silver ion solution that causes metal silver to precipitate on a metal label by physical development, a solution of a phenylenediamine compound and a naphthol compound which serves as a colorant due to an action of a peroxidase label and hydrogen peroxide, and the like.

For details, a so-called developer can be used as an amplification liquid containing an amplification reagent, where developers are described in general books in the field of photographic chemistry (for example, "Fundamentals of Photograph Engineering (Revised) -Silver Halide Photography-" (edited by the Society of Photography and Imaging of Japan, Corona Publishing Co., Ltd.), "Chemistry of Photography" (by Akira SASAI, Shashinkogyo Publishing Company), and "Latest Prescription Handbook" (by Shinichi KIKUCHI et al., Amiko Publishing Company)). It is possible to use any developer as an amplification liquid without particular limitation as long as it is a so-called physical developer which contains silver ions in the liquid and in which the silver ions in the liquid are reduced mainly by metal colloids and the like which form the core of development.

In the present invention, two kinds of amplification reagents are used. It is preferable that a first amplification reagent be incorporated in a first amplification liquid, and a second amplification reagent be incorporated in a second amplification liquid among the two kinds of amplification reagents used for amplifying signals of label substances trapped in the label substance-trapping region, and amplification be performed by adding the first amplification liquid and the second amplification liquid in this order. The first amplification liquid is preferably added to a pad which is for liquid sending of a reducing agent solution and is located in an upstream direction of a label substance-holding pad and a specimen-added pad.

As a specific example of the amplification liquid, it is possible to use a combination of a first amplification liquid containing a reducing agent capable of reducing silver ions and a second amplification liquid containing a compound containing silver.

Hereinafter, the reducing agent capable of reducing silver ions contained in the first amplification liquid, and the compound containing silver contained in the second amplification liquid will be described.

### 7-1. Compound containing silver

As the compound containing silver, it is possible to use silver ion-containing compounds such as organic silver salts, inorganic silver salts, or silver complexes. The compound is preferably a silver ion-containing compound having a high solubility in solvents such as water, and examples thereof include silver nitrate, silver acetate, silver lactate, silver butyrate, silver thiosulfate, and the like. Silver nitrate is particularly preferred. Silver complexes are preferably silver complexes coordinated with ligands having a water-soluble group such as a hydroxyl group or a sulfone group, and examples thereof include silver hydroxythioether and the like.

In general, as silver, inorganic silver salts or silver complexes are contained in an amount of 0.001 mol/m² to 0.2 mol/m², and are preferably contained in an amount of 0.01 mol/m² to 0.05 mol/m².

### 7-2. Reducing agent capable of reducing silver ions

As the reducing agent capable of reducing silver ions, any inorganic or organic material or a mixture thereof can be used as long as it can reduce silver ions to silver.

Preferred examples of inorganic reducing agents include reducing metal salts and reducing metal complex salts which are capable of changing an atomic value with metal ions such as Fe²⁺, V²⁺, or Ti³⁺. In a case in which an inorganic reducing agent is used, it is necessary to remove or detoxify oxidized ions by complexing or reducing the oxidized ions. For example, in a system in which Fe²⁺ is used as a reducing agent, a complex of Fe³⁺, which is an oxide, can be formed using citric acid or EDTA and detoxified. In the present system, such an inorganic reducing agent is preferably used, and a metal salt of Fe²⁺ is more preferred.

It is also possible to use a main developing agent used in a light-sensitive silver halide photographic material of a wet-type (such as methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco colorants), and other materials obvious to those who are skilled in the technology in the present field, such as a material disclosed in US6020117A.

As the reducing agent, an ascorbic acid reducing agent is also preferable. Useful ascorbic acid reducing agents include ascorbic acid and analogs thereof, and isomers and derivatives thereof. Preferred examples thereof include D- or L-ascorbic acid and sugar derivatives thereof (such as γ-lactoascorbic acid, glucoascorbic acid, fucoascorbic acid, glucoheptoascorbic acid, and maltoascorbic acid), a sodium salt of ascorbic acid, a potassium salt of ascorbic acid, isoascorbic acid (or L-erythroascorbic acid), a salt thereof (such as alkali metal salts, ammonium salts, or salts known in the technical field), ascorbic acid of the enediol type, ascorbic acid of the enaminol type, ascorbic acid of the thioenol type, and the like. Particularly preferred examples thereof include D-, L-, or D,L-ascorbic acid (or an alkali metal salt thereof) or isoascorbic acid (or an alkali metal salt thereof), and a sodium salt is a preferable salt. A mixture of these reducing agents can be used as necessary.

### 8. Other auxiliaries

As other auxiliaries of the amplification liquid, buffers, preservatives such as antioxidants or organic stabilizers, and rate regulators may be included. As the buffer, it is possible to use, for example, acetic acid, citric acid, sodium hydroxide, or salts of these substances, or a buffer formed of tris(hydroxymethyl)aminomethane or other buffers used in general chemical experiments. A pH can be adjusted to an optimum pH for the amplification liquid by appropriately using these buffers. Furthermore, an alkylamine can be used as an additive as an antifogging agent, and dodecylamine is particularly preferable. In addition, a surfactant can be used in order to improve solubility of these additives, and C₉H₁₉-C₆H₄-O-(CH₂CH₂O)₅₀H is particularly preferable.

As a method of spotting the amplification reagent on the chromatographic kit, a method is preferable in which a reducing agent solution as the first amplification liquid is spotted on a pad for liquid sending of the reducing agent solution, a silver ion solution as the second amplification liquid is spotted from above on a region including a label substance-trapping region, and the silver ion solution is infiltrated in a thickness direction of a porous carrier.

As a method of incorporating the two kinds of amplification reagents into the chromatographic kit, there is a method of disposing a pot containing a solution containing each of the amplification reagents above a site at which each of the amplification reagents is spotted. It is preferable that the reducing agent solution (first amplification liquid) be placed on the pad for liquid sending of the reducing agent solution, and the pot containing the silver ion solution (second amplification liquid) be placed immediately above a hole filled with the silver ion solution. By disposing in this manner, the liquid flows by pushing each pot and can be spotted on a predetermined site.

### 9. Chromatographic kit

In the chromatographic kit of the embodiment of the present invention, the label substance modified with the first binding substance for the test substance may be provided on the porous carrier in advance, or alternatively, the label substance modified with the first binding substance for the test substance may be provided separately from the porous carrier. In this case, the label substance modified with the first binding substance for the test substance, which is provided separately from the porous carrier, can be measured by a method such as a method of mixing the label substance with a test specimen and then spreading the mixture on the porous carrier.

The chromatographic kit of the embodiment of the present invention further includes the compound containing silver and the reducing agent capable of reducing silver ions.

The chromatographic kit of the embodiment of the present invention may include a housing case including, therein, the porous carrier having a reaction site, the compound containing silver, and the reducing agent capable of reducing silver ions.

The chromatographic kit of the embodiment of the present invention may further include pots each having a tearable member, in which the compound containing silver and the reducing agent capable of reducing silver ions may be respectively sealed in the pots. In this case, the pots can be broken by an external force.

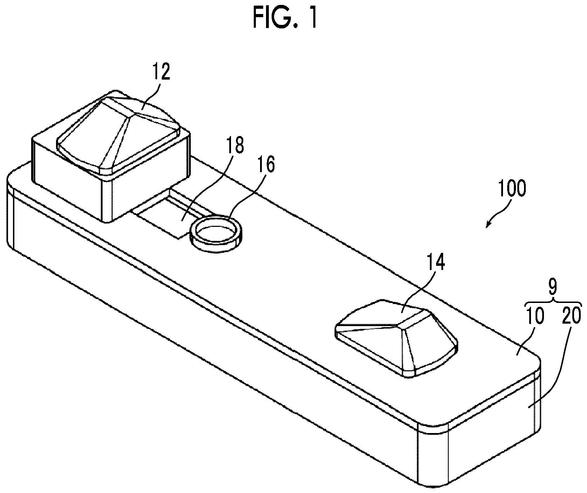

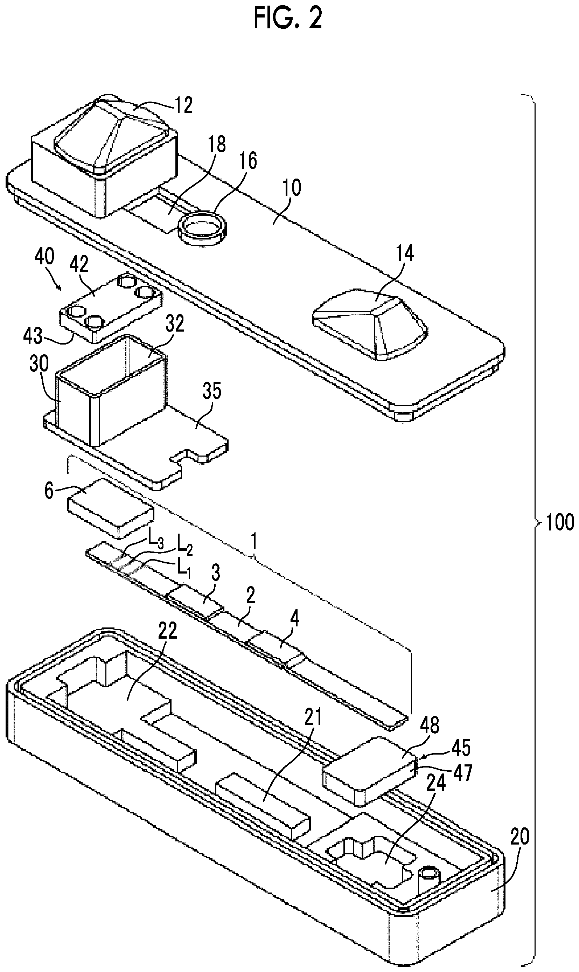

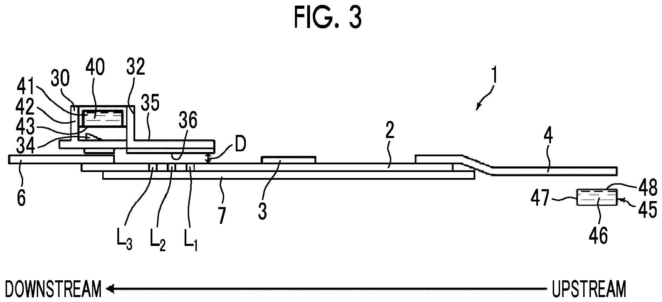

Fig. 1 is an exploded schematic perspective view illustrating a chromatographic kit 100 showing an example of the present invention, and Fig. 2 is an exploded schematic perspective view of the chromatographic kit 100 of Fig. 1. Fig. 3 is a schematic side view showing a positional relationship between an inspection strip, and a first pot and a second pot.

As illustrated in Fig. 1 to Fig. 3, in the chromatographic kit 100 of the present embodiment, a housing case 9 includes an inspection strip 1 that has a porous carrier 2 having an inspection region of a test substance and is for spreading a specimen liquid, and a first pot 40 and a second pot 45 which are for amplifying a detection signal in the inspection region, which respectively include a surface having a sheet member, and in which a first amplification liquid 41 and a second amplification liquid 46 are sealed, respectively. The housing case 9 includes a lower case 20 having an accommodation portion 21 in which the inspection strip 1 is disposed, an upper case 10 joined to the lower case 20 along a peripheral edge, and a middle member 30 disposed between the upper case 10 and the lower case 20. In explaining the chromatographic kit 100 of the present embodiment, the upper case 10 side is defined as an upper part and the lower case 20 side is defined as a lower part.

The middle member 30 has a first pot accommodation portion 32 which accommodates the first pot 40 and which has, on a bottom surface, an amplification liquid-filled hole for dropwise addition of the first amplification liquid 41 onto the porous carrier 2. In addition, a protrusive tearing portion 34 that tears up a sheet member 43 is provided at a location facing the sheet member 43 of the first pot 40 in the first pot accommodation portion 32. In the present example, the first pot 40 is disposed above the first pot accommodation portion 32 so that the surface having the sheet member 43 is the lower surface, and the tearing portion 34 is provided on the bottom surface of the first pot accommodation portion 32 facing the sheet member 43 (refer to Fig. 3).

In addition, a flow path-forming portion 35 is provided so as to extend to a downstream side of the bottom surface of the first pot accommodation portion 32 of the middle member 30. The flow path-forming portion 35 is disposed to correspond with positions above an inspection region L₁, a confirmation region L₂, and an amplification label region L₃, and is formed of a transparent material so that these regions L₁ to L₃ can be visually checked.

The upper case 10 includes, on a part facing the first pot 40, a first protrusive deforming portion 12 that is deformed towards the first pot 40 side so as to allow the tearing portion 34 of the middle member 30 to tear up the sheet member 43 of the first pot 40 by application of a pressing force from the outside. In addition, the upper case 10 includes, on a part facing the second pot 45, a second protrusive deforming portion 14 that is deformed towards the second pot 45 side so that a sheet member 48 of the second pot 45 is torn up by application of a pressing force from the outside.

In addition, a hole 16 for dropwise addition of a specimen liquid is provided on the upper case 10, and a specimen liquid is added dropwise onto a label-holding pad 3 of the inspection strip 1 from this hole 16. In a case where a location of the label-holding pad 3 is adjusted so that locations of the hole 16 and the label-holding pad 3 correspond to each other, it is possible to reliably spot the specimen liquid onto the label-holding pad 3. In addition, the upper case 10 includes an observation window 18 for visually checking the three regions L₁ to L₃ at positions corresponding to the flow path-forming portion 35 of the middle member 30.

In the lower case 20, as an accommodation portion in which the inspection strip 1 is disposed, a porous carrier accommodation portion 21 on which the porous carrier 2 is mounted is provided, and an absorption pad accommodation portion 22 on which an absorption pad 6 is mounted is provided on the downstream side of the porous carrier accommodation portion. In addition, a second pot accommodation portion 24 in which the second pot 45 is accommodated is provided on the upstream side of the porous carrier accommodation portion 21.

Fig. 3 is a schematic side view illustrating a positional relationship between the inspection strip 1, the middle member 30, and the two pots 40 and 45. As illustrated in Fig. 3, the inspection strip 1 includes the porous carrier 2 spreading the specimen liquid, the label-holding pad 3 having a label substance modified with an antibody immobilized on the porous carrier 2, a liquid-sending pad 4 which is disposed in contact with one end of the porous carrier 2 and sends the second amplification liquid 46 to the porous carrier 2, and the absorption pad 6 disposed in contact with the other end of the porous carrier 2. The porous carrier 2 is immobilized to and supported by a back pressure-sensitive adhesion sheet 7. In addition, between the label-holding pad 3 and the absorption pad 6, the porous carrier 2 has the inspection region L₁, the confirmation region L₂, and the amplification label region L₃ in this order from the label-holding pad 3 side.

In the present specification, there are cases in which the porous carrier 2, which has the inspection region L₁, the confirmation region L₂, and the amplification label region L₃ formed thereon, is referred to as a chromatographic carrier. In addition, in the present specification, as illustrated in Fig. 3, the liquid-sending pad 4 side is defined as an upstream side and the absorption pad 6 side is defined as a downstream side.

The middle member 30 is positioned at the upper portion and the downstream end side of the inspection strip 1, and the first pot 40 is disposed in the first pot accommodation portion 32 of the middle member 30 with the sheet member 43 facing downward. The second pot 45 is accommodated at the lower portion and the upstream end of the inspection strip 1 in the lower case 20 with the sheet member 48 facing upward.

As shown in Fig. 3, a gap (clearance) D is formed between a rear surface 36 of the flow path-forming portion 35 of the middle member 30, and the porous carrier 2 of the inspection strip 1. The gap D is preferably within a range of 0.01 mm to 1 mm. In a case where the gap is 0.01 mm or more, an amplification liquid and the like can be sufficiently infiltrated, and in a case where the gap is 1 mm or less, capillary force is exerted and the gap between the porous carrier 2 and the middle member 30 can be uniformly filled with the first amplification liquid 41.

In the first pot 40 in which the first amplification liquid 41 is sealed, for example, a container 42 which is formed of a resin material and has an opening on one surface is filled with the first amplification liquid 41, and the opening of the container 42 is covered with the tearable sheet member 43 and sealed.

Similarly, in the second pot 45 in which the second amplification liquid 46 is sealed, for example, a container 47 which is formed of a resin material and has an opening on one surface is filled with the second amplification liquid 46, and the opening of the container 47 is covered with the tearable sheet member 48 and sealed.

As the tearable sheet members 43 and 48 in the first pot 40 and the second pot 45, laminate films such as aluminum foils and aluminum sheets are suitability used. The term "tear" refers to a state in which the sheet does not regenerate after being torn up.

### 10. Method of calculating average particle size of label substance in detection

In detection (after amplification), a test line portion is cut out, a rear surface of a specimen is attached to a specimen stand with carbon paste, and thereafter, a cross section is cut, carbon vapor deposition is performed, and a shape and a size are observed with a scanning electron microscope. For example, observation of a specimen surface by reflected electrons at an accelerating voltage of 10 KV using a FE-STEM S-5500 manufactured by Hitachi High-Technologies Corporation can be performed with a scanning electron microscope (SEM). Thereafter, 100 signal particles are selected, a circle equivalent diameter of a projection area of the particles is measured, and an average value is calculated and used for an average particle size in the detection.

An average particle size of the label substance in the detection is preferably 1 µm or more and 20 µm or less, and more preferably 3 µm or more and 20 µm or less.

Hereinafter, the present invention will be described more specifically with reference to Examples of the present invention. In the following Examples, materials, amounts used, ratios, details of a treatment, treatment procedures, and the like may be appropriately changed without departing from the gist of the present invention. Accordingly, the scope of the present invention should not be limitedly interpreted by the following specific examples.

### Examples

Hereinafter, examples and comparative examples of the chromatographic kit of the present invention will be described. Chromatographic kits of the examples and the comparative examples are chromatographic kits for influenza virus antigen detection for detecting influenza virus antigens as a test substance.

### (1) Production of chromatographic kit

### (1-1) Preparation of antibody-modified gold colloid

1 mL of 50 mmol/L KH₂PO₄ buffer (pH 7.5) was added to 9 mL of a solution (Product No.: EM. GC50, manufactured by Boston Biomedical Inc.) containing gold colloid having a diameter of 50 nm so as to adjust a pH. Thereafter, 1 mL of a solution containing 160 mg/mL of an anti-influenza A-type monoclonal antibody (Anti-Influenza A SPTN-5 7307, manufactured by Medix Biochemica) was added thereto and stirred for 10 minutes. Thereafter, after the solution mixture was left to stand for ten minutes, 550 µL of an aqueous solution containing 1 mass% polyethylene glycol (PEG; weight-average molecular weight (Mw.): 20,000, Product No. 168-11285, manufactured by FUJIFILM Wako Pure Chemical Corporation) was added to the solution mixture and stirred for 10 minutes. Subsequently, 1.1 mL of an aqueous solution of 10 mass% bovine serum albumin (BSA; Fraction V, Product No.: A-7906, manufactured by Sigma-Aldrich Co. LLC.) was added thereto and stirred for 10 minutes. This solution was centrifuged for 30 minutes under conditions of 8,000 × g at 4°C using a centrifugal separator (himacCF16RX, manufactured by Hitachi Ltd.). The supernatant liquid was removed with 1 mL thereof remaining at the bottom of a container, and gold colloid contained in the 1 mL solution remaining at the bottom of the container was re-dispersed by an ultrasonic washer. Thereafter, the solution was dispersed in 20 mL of a gold colloid preservative solution (20 mmol/L Tris-HCl (tris hydrochloric acid) buffer (pH 8.2), 0.05% PEG (Mw: 20,000), 150 mmol/L NaCl, 1% BSA), and was centrifuged again under the same conditions using the same centrifugal separator. Thereafter, the supernatant liquid was removed, ultrasonic dispersion was performed, and then the solution was dispersed in the gold colloid preservative solution. Thereby, an anti-influenza A-type antibody-modified gold colloid (50 nm) solution was obtained as a label substance modified with an antibody, which is a first substance capable of binding to a test substance.

### (1-2) Production of label-holding pad

The anti-influenza A-type antibody-modified gold colloid produced in (1-1) was diluted with water so that a concentration of a Tris-HCl buffer (pH: 8.2) reached 20 mmol/L, a concentration of PEG (Mw: 20,000) reached 0.05 mass%, a concentration of sucrose reached 5 mass%, and an optical density of the gold colloid at 520 nm reached 0.2 in a case where an optical path length was set to 10 mm, and thereby a gold colloid coating liquid was obtained. This coating liquid was uniformly applied onto glass fiber pads each cut into 5 mm x 300 mm (Glass Fiber Conjugate Pad, manufactured by EMD Millipore Corporation) by 0.1 mL per pad, and was dried at reduced pressure for 24 hours. Thereby an anti-influenza A-type antibody-modified gold colloid-holding pad was obtained.

### (1-3) Production of chromatographic carrier

Using nitrocellulose membrane cut into 60 mm x 300 mm (with a plastic backing, HiFlow Plus HF135 (capillary flow rate = 135 sec/cm), manufactured by EMD Millipore Corporation) as a porous carrier, an inspection region, a confirmation region, and an amplification label region were formed on this membrane by a method described below, and thereby a chromatographic carrier was produced.

An anti-influenza A-type monoclonal antibody (Anti-Influenza A SPTN-5 7307, manufactured by Medix Biochemica) solution, which was prepared so that a concentration was 1.5 mg/mL, was applied in a line shape at a position 15 mm from the downstream side of a 60 mm short side of the nitrocellulose membrane, and thereby the inspection region was produced. Furthermore, an anti-mouse IgG antibody (anti-mouse IgG(H+L), rabbit F(ab')₂, Product No. 566-70621, manufactured by FUJIFILM Wako Pure Chemical Corporation) solution, which was prepared so that a concentration was 0.2 mg/mL, was applied in a line shape at a position 11 mm from the downstream side of the 60 mm short side, and thereby the confirmation region was obtained. Furthermore, a Bromocresol Green (manufactured by FUJIFILM Wako Pure Chemical Corporation), which was prepared so that a concentration was 30 mmol/L, was applied in a line shape at a position 9 mm from the downstream side of the 60 mm short side, and thereby the amplification label region was obtained. After the application of the respective solutions, the nitrocellulose membrane was dried at 50°C for 30 minutes using a warm air-type dryer. After completion of the drying, the nitrocellulose membrane dried as described above was immersed in a vat in which 500 mL of a blocking liquid (50 mmol/L of a boric acid buffer (pH: 8.5) containing 0.5 mass% casein (derived from milk, Product No. 030-01505, manufactured by FUJIFILM Wako Pure Chemical Corporation)) was put, and the membrane was left to stand for 30 minutes. Thereafter, the nitrocellulose membrane was taken out, the nitrocellulose membrane was immersed in 500 mL of a washing and stabilizing liquid (50 mmol/L Tris-HCl buffer (pH: 7.5) containing 0.5 mass% sucrose and 0.05 mass% sodium cholate) prepared in another vat, and the membrane was left to stand for 30 minutes. Thereafter, the nitrocellulose membrane was removed from the liquid and dried at an environment of 25°C for 24 hours.

A part to which the anti-influenza A-type antibody was immobilized corresponded to the inspection region including the second substance binding to the test substance, a part to which the anti-mouse IgG antibody was immobilized corresponded to the confirmation region including the substance that can bind to the first substance, and a part to which the bromocresol green was immobilized corresponded to the amplification label region including the substance reacting with the first amplification liquid.

### (1-4) Production of inspection strip

The chromatographic carrier produced in (1-3) was attached to a back pressure-sensitive adhesion sheet (60 mm × 300 mm (manufactured by Nippon Flour Mills Co., Ltd.)). Next, a 3 mm wide double-sided tape (NITTO DENKO CORPORATION) was fixed at a position 26 mm from the downstream side of a short side of the chromatographic carrier. Thereafter, the gold colloid-holding pad was fixed to the chromatographic carrier so that the downstream end of the double-sided tape and the downstream end of the glass fiber pad (Glass Fiber Conjugate Pad, manufactured by EMD Millipore Corporation) cut into 8 mm × 300 mm overlapped each other. A liquid-sending pad (glass fiber pad cut into 25 mm × 300 mm (Glass Fiber Conjugate Pad, manufactured by EMD Millipore Corporation) was attached to the upstream side of the chromatographic carrier so that the liquid-sending pad and the chromatographic carrier overlapped each other by 7 mm. Using a guillotine style cutter (CM4000, manufactured by NTC/NIPPN TechnoCluster, Inc.)), a member produced in this manner was cut parallel to a direction perpendicular to a 300 mm long side such that a width was 5 mm. Thereby, 60 inspection strips (however, no absorption pads were included) were produced.

### (1-5) Production of amplification liquid

### (1-5-1) Production of amplification liquid (reducing agent solution) sealed in second pot

23.6 mL of an aqueous solution of 1 mol/L iron nitrate produced by dissolving iron (III) nitrate nonahydrate (manufactured by FUJIFILM Wako Pure Chemical Corporation, 095-00995) in water, and 13.1 g of citric acid (manufactured by FUJIFILM Wako Pure Chemical Corporation, 038-06925) were dissolved in 290 g of water. After the substances were fully dissolved, 36 mL of a nitric acid (10 mass%) solution was added thereto while stirring the solution using a stirrer, and 60.8 g of iron (II) ammonium sulfate hexahydrate (manufactured by FUJIFILM Wako Pure Chemical Corporation, 091-00855) was added thereto. The solution thus prepared was used for a reducing agent solution which was the second amplification liquid sealed in the second pot.

### (1-5-2) Production of amplification liquid (silver ion solution) sealed in first pot

8 mL of a silver nitrate solution (including 10 g of silver nitrate) and 24 mL of an aqueous solution of 1 mol/L iron nitrate were added to 66 g of water. Furthermore, this solution was mixed with a solution obtained by dissolving 5.9 mL of nitric acid (10 mass%), 0.1 g of dodecylamine (manufactured by FUJIFILM Wako Pure Chemical Corporation, 123-00246), and 0.1 g of a surfactant C₁₂H₂₅-C₆H₄-O-(CH₂CH₂O)₅₀H in 47.6 g of water in advance. This mixed solution was used for a silver ion solution which was the first amplification liquid sealed in the first pot.

### (1-6) Production of absorption pad

60 glass fiber pads each cut into 12 mm × 10 mm (glass filter paper, manufactured by Advantech Co., Ltd.) were prepared and used for absorption pads.

### (1-7) Production of components of chromatographic kit

The lower case 20, the upper case 10, the middle member 30, the first pot 40, and the second pot 45 constituting the chromatographic kit 100 shown in Fig. 1 were respectively produced by injection molding using polypropylene as a material. The upper case was produced by injection molding using, as a material, polypropylene containing 50 mass% of TAFTHREN (registered trademark) which is an olefinic elastomer manufactured by Sumitomo Chemical Co., Ltd. The upper case 10 has two deformable portions (first protrusive deforming portion and second protrusive deforming portion). These two deforming portions are parts not separated from the upper case 10, and the upper case was produced by injection molding with the entire boundary as a part of the upper case 10.

In the upper case, the first protrusive deforming portion 12 shown in Figs. 1 and 2 and the like has two protrusion portions, and the second protrusive deforming portion 14 has one protrusion portion.

### (1-8) Production of chromatographic kit

The lower case 20, the inspection strip produced in (1-4), and the absorption pad 6 produced in (1-6) were fixed as illustrated in Figs. 1 and 2. Next, the first pot 40 and the second pot 45 were respectively filled with the first amplification liquid 41 to be sealed in the first pot 40 produced in (1-5-2) and the second amplification liquid 46 to be sealed in the second pot 45 produced in (1-5-1). The second pot 45 was sealed with aluminum foil as the sheet member 48, and the first pot 40 was sealed with aluminum foil as the sheet member 43. As shown in Figs. 1 and 2, the second pot 45 was attached to the lower case 20 with the sheet member 48 facing upward, and the first pot 40 was attached to the middle member 30 with the sheet member 43 facing downward. In addition, in a state in which the upper case 10 and the lower case 20 were fitted so that the outer peripheries thereof came in contact with each other, contact portions of the upper case and the lower case were joined by ultrasonic welding. In this case, it was confirmed that the entire portions of the welded portions were uniformly welded in a sealed state. The chromatographic kit of Example 1 was produced in this manner.

### (1-9) Production of spread liquid

A spread liquid of 400 mmol/L Tricine buffer solution (pH: 8.5) containing 0.1 mass% casein and 1 mass% Tween (registered trademark) 40 was prepared, and 100 µL thereof was added to a 1.5 mL tube. This was used for a reagent for treating test samples such as nasal discharge, sputum, a pharyngeal swab, urine, and the like.

Because 100 µL of the test sample was used in a case of evaluation, final concentrations of each of the substances contained in the spread liquid in the case of spreading were 0.05 mass% casein, 0.5 mass% Tween 40, and 200 mmol/L Tricine buffer solution (pH 8.5).

### (2) Evaluation

### (2-1) Spotting

Using phosphate buffered saline (PBS) containing nasal discharge from a healthy subject, 100 µL of a liquid, which was prepared by diluting commercially available inactivated influenza A-type virus such that a protein concentration was 1.0 ng/mL, was added into and dissolved in the reagent produced in (1-9). The resultant product was used for a positive test specimen. In addition, 100 µL of PBS containing nasal discharge of a healthy subject and not containing inactivated influenza A-type virus was added into and dissolved in the reagent produced in (1-9). The resultant product was used for a negative test specimen. The anti-influenza A-type antibody-modified gold colloid-holding pad produced in (1-2) was cut into 5 mm × 4 mm. The cut pad was added into these positive test specimen and negative test specimen, and left still for 120 minutes. After standing still, 40 µL of each of the liquids was spotted.

### (2-2) Spreading of amplification liquid (reducing agent solution) sealed in second pot

30 minutes after spotting the specimen liquid of (2-1), the aluminum foil, which is the sheet member 48 sealing the second amplification liquid 46 sealed in the second pot 45, was torn up by pressing down the second protrusive deforming portion 14, the liquid-sending pad 4 was immersed in the second pot 45, and thereby the second amplification liquid 46 was supplied to the porous carrier 2 utilizing capillarity.

### (2-3) Silver amplification

After discoloration of the amplification label region L₃ from green to orange, the first protrusive deforming portion 12 (first protrusive deforming portion 114 in Example 2) was pressed down to move the first pot 40 toward the tearing portion 34 of the pot accommodation portion 32 of the middle member 30, and thereby the tearing portion 34 was caused to press and tear up the aluminum foil which is the sheet member 43 sealing the first pot 40. The silver ion solution which is the first amplification liquid 41 was supplied to the porous carrier 2 from the opening portion of the middle member 30, and thereby a silver amplification reaction was caused. The silver amplification reaction was completed in tens of seconds.

### (2-4) Calculation of concentration value of inspection region

Concentrations in the inspection region L₁ and the confirmation region L₂ of the chromatographic kit of Example 1 that had been subjected to the amplification treatment were visually observed. Furthermore, the chromatographic carrier was taken out from the chromatographic kit of Example 1, a concentration of the inspection region L₁ was imaged using LAS4000 (manufactured by Fujifilm Corporation), and a concentration difference (ΔOD = (concentration of inspection region L₁) - (concentration of background portion)) was calculated. It can be said that amplification results are generally favorable in a case of ΔOD ≥ 0.020.

Table 3 shows results of evaluation results.

### [Example 2: Tween 60]

Measurement was performed in the same manner as in Example 1 except that 1 mass% Tween 60 was used instead of 1 mass% Tween 40 in (1-9) of Example 1. A final concentration contained in the spread liquid in the case of spreading was 0.5 mass% Tween 60. Table 3 shows results of evaluation results.

### [Example 3: Tween 80]

Measurement was performed in the same manner as in Example 1 except that 1 mass% Tween 80 was used instead of 1 mass% Tween 40 in (1-9) of Example 1. A final concentration contained in the spread liquid in the case of spreading was 0.5 mass% Tween 80. Table 3 shows results of evaluation results.

### [Comparative Example 1: no surfactant]

Measurement was performed in the same manner as in Example 1 except that 1 mass% Tween 40 in (1-9) of Example 1 was not contained. Table 3 shows results of evaluation results.

### [Comparative Example 2: BSA]

Measurement was performed in the same manner as in Example 1 except that 0.1 mass% BSA was used instead of 0.1 mass% casein in (1-9) of Example 1. A final concentration contained in the spread liquid in the case of spreading was 0.05 mass% BSA. Table 3 shows results of evaluation results.

Average particle sizes of labeled particles that had been subjected to the amplification treatment were calculated using a scanning electron microscope (FE-STEM S-5500 manufactured by Hitachi High-Technologies Corporation), and all average particle sizes were found to be 10 µm.

Table 3 shows results of evaluating the same test for each of the examples.
ΔOD was determined according to the following criteria.
+: 0.020 or more (visible region)
-: less than 0.020 (invisible region)

A numerical value in parentheses in the column of the negative test specimen in the table indicates ΔOD.

**[Table 3]**

| | Surfactant | Protein | Buffer | Negative test specimen |
|---|---|---|---|---|
| Example 1 | Tween 40 (0.5 mass%) | Casein (0.05 mass%) | Tricine (200 mmol/L) | - (0.009) |
| Example 2 | Tween 60 (0.5 mass%) | Casein (0.05 mass%) | Tricine (200 mmol/L) | - (0.003) |
| Example 3 | Tween 80 (0.5 mass%) | Casein (0.05 mass%) | Tricine (200 mmol/L) | - (0.003) |
| Comparative Example 1 | Not contained | Casein (0.05 mass%) | Tricine (200 mmol/L) | + (0.034) |
| Comparative Example 2 | Tween 40 (0.5 mass%) | BSA (0.05 mass%) | Tricine (200 mmol/L) | + (0.097) |

As described above, in Examples 1 to 3, by using the spread liquid containing Tricine, casein, and the nonionic surfactants (Tween 40, Tween 60, and Tween 80), results, in which false positives due to a non-specific reaction were not generated, were obtained for the negative test specimen having a concentration of 0 ng/mL.

On the other hand, in the case (Comparative Example 1) in which the measurement was performed in the same manner as in Example 1 except that Tween 40 in Example 1 was not contained, false positives due to a non-specific reaction were generated for the negative test specimen having a concentration of 0 ng/mL, and therefore it could not be used for an inspection kit.

On the other hand, in the case (Comparative Example 2) in which the measurement was performed in the same manner as in Example 1 except that BSA was used instead of casein, false positives due to a non-specific reaction were generated for the negative test specimen having a concentration of 0 ng/mL, and therefore it could not be used for an inspection kit.

In addition, it was confirmed that visible results were obtained in any of the examples with the positive test specimen having a concentration of 1.0 ng/mL.

Next, spread liquids containing various concentrations of Tween 40 were produced to perform detection and measurement of influenza virus (Examples 4 and 5).

### [Example 4: concentration of Tween 40 is 5 mass%]

Measurement was performed in the same manner as in Example 1 except that 5 mass% Tween 40 was used instead of 1 mass% Tween 40 in (1-9) of Example 1. A final concentration contained in the spread liquid in the case of spreading was 2.5 mass% Tween 40. Table 4 shows results of evaluation results.

### [Example 5: concentration of Tween 40 is 0.1 mass%]

Measurement was performed in the same manner as in Example 1 except that 0.1 mass% Tween 40 was used instead of 1 mass% Tween 40 in (1-9) of Example 1. A final concentration contained in the spread liquid in the case of spreading was 0.05 mass% Tween 40. Table 4 shows results of evaluation results.

Table 4 shows results of evaluating the same test for each of the examples.
ΔOD was determined according to the following criteria.
+: 0.020 or more (visible region)
-: less than 0.020 (invisible region)

A numerical value in parentheses in the column of the negative test specimen in the table indicates ΔOD.

**[Table 4]**

| | Surfactant | Protein | Buffer | Negative test specimen |
|---|---|---|---|---|
| Example 1 | Tween 40 (0.5mass%) | Casein (0.05 mass%) | Tricine (200 mmol/L) | - (0.009) |
| Example 4 | Tween 40 (2.5 mass%) | Casein (0.05 mass%) | Tricine (200 mmol/L) | - (0.007) |
| Example 5 | Tween 40 (0.05 mass%) | Casein (0.05 mass%) | Tricine (200 mmol/L) | - (0.019) |

Based on these results, it was confirmed that even in the case in which the final concentration of Tween 40 was changed to 2.5 mass% and 0.05 mass% in Examples 4 and 5, false positives due to a non-specific reaction were not generated for the negative test specimen having a concentration of 0 ng/mL, which was the same result as in Example 1.

Next, spread liquids were produced using various buffers to perform detection and measurement of influenza virus. Concentrations of casein and Tween 40 were measured in the same manner as in Example 1 (Examples 6 to 9 and Comparative Examples 3 and 4). Table 5 shows results of evaluation results.

### [Example 6: Bicine] (comparative example)

Measurement was performed in the same manner as in Example 1 except that 400 mmol/L Bicine (pH 8.5) was used instead of 400 mmol/L Tricine (pH 8.5) in (1-9) of Example 1. A final concentration contained in the spread liquid in the case of spreading was 200 mmol/L Bicine. Table 5 shows results of evaluation results.

### [Example 7: HEPPSO]

Measurement was performed in the same manner as in Example 1 except that 400 mmol/L HEPPSO (pH 8.5) was used instead of 400 mmol/L Tricine (pH 8.5) in (1-9) of Example 1. A final concentration contained in the spread liquid in the case of spreading was 200 mmol/L HEPPSO. Table 5 shows results of evaluation results.

### [Example 8: TAPS]

Measurement was performed in the same manner as in Example 1 except that 400 mmol/L TAPS (pH 8.5) was used instead of 400 mmol/L Tricine (pH 8.5) in (1-9) of Example 1. A final concentration contained in the spread liquid in the case of spreading was 200 mmol/L TAPS. Table 5 shows results of evaluation results.

### [Example 9: CHES]

Measurement was performed in the same manner as in Example 1 except that 400 mmol/L CHES (pH 8.5) was used instead of 400 mmol/L Tricine (pH 8.5) in (1-9) of Example 1. A final concentration contained in the spread liquid in the case of spreading was 200 mmol/L CHES. Table 5 shows results of evaluation results.

### [Comparative Example 3: glycine]

Measurement was performed in the same manner as in Example 1 except that 400 mmol/L glycine (pH 8.5) was used instead of 400 mmol/L Tricine (pH 8.5) in (1-9) of Example 1. A final concentration contained in the spread liquid in the case of spreading was 200 mmol/L glycine. Table 5 shows results of evaluation results.

### [Comparative Example 4: boric acid]

Measurement was performed in the same manner as in Example 1 except that 400 mmol/L boric acid (pH 8.5) was used instead of 400 mmol/L Tricine (pH 8.5) in (1-9) of Example 1. A final concentration contained in the spread liquid in the case of spreading was 200 mmol/L boric acid. Table 5 shows results of evaluation results.

Table 5 shows results of evaluating the same test for each of the examples.
ΔOD was determined according to the following criteria.
++: 0.040 or more (fully visible region)
+: 0.020 or more (visible region)
-: less than 0.020 (invisible region)

A numerical value in parentheses in the columns of the negative test specimen and the positive test specimen in the table indicates ΔOD.

**[Table 5]**

| | Surfactant | Protein | Buffer | Negative test specimen | Positive test specimen |
|---|---|---|---|---|---|
| Example 1 | Tween 40 (0.5 mass%) | Casein (0.05 mass%) | Tricine (200 mmo/L) | - (0.007) | ++ (0.049) |
| Example 6 | Tween 40 (0.5 mass%) | Casein (0.05 mass%) | Bicine (200 mmo/L) | - (0.006) | ++ (0.046) |
| Example 7 | Tween 40 (0.5 mass%) | Casein (0.05 mass%) | HEPPSO (200 mmo/L) | - (0.000) | ++ (0.041) |
| Example 8 | Tween 40 (0.5 mass%) | Casein (0.05 mass%) | TAPS (200 mmo/L) | - (0.003) | ++ (0.044) |
| Example 9 | Tween 40 (0.5 mass%) | Casein (0.05 mass%) | CHES (200 mmo/L) | - (0.001) | ++ (0.046) |
| Comparative Example 3 | Tween 40 (0.5 mass%) | Casein (0.05 mass%) | Glycine (200 mmo/L) | - (0.000) | + (0.030) |
| Comparative Example 4 | Tween 40 (0.5 mass%) | Casein (0.05 mass%) | Boric acid (200 mmo/L) | - (0.003) | + (0.028) |

Based on these results, it was confirmed that even in the case in which a buffer was changed to other Good's buffers in Examples 6 to 9, false positives due to a non-specific reaction were not generated for the negative test specimen having a concentration of 0 ng/mL, and the region was visible in the case of the positive test specimen having a concentration of 1.0 ng/mL. Example 6 is for comparison reasons only.

On the other hand, in the case in which Tricine was changed to glycine and a borate buffer which was not other Good's buffers in Comparative Examples 3 and 4, results were obtained, in which false positives due to a non-specific reaction were not generated for the negative test specimen having a concentration of 0 ng/mL as in Example 1, but there was a decrease of 36% in average with respect to ΔOD of Examples 1 and 6 to 9 for the case of the positive test specimen having a concentration of 1.0 ng/mL. Based on this result, it was found that the Good's buffer selected from Tricine, HEPPSO, TAPS and CHES is desirable for a buffer of the spread liquid.

Next, a protein concentration of the diluent solution of influenza virus of (2-1) was detected and measured at 0 ng/mL, 0.1 ng/mL, and 1.0 ng/mL. Measurement was performed using the same type of buffer and surfactant, and the same concentration of casein other than a concentration of the surfactant as in Example 1 (Example 10 and Comparative Examples 5 and 6). Table 6 shows results of evaluation results.

### [Example 10: Bicine and Tween 60] (comparative example)

Measurement was performed in the same manner as in Example 1 except that 400 mmol/L Bicine (pH 8.5) and 1 mass% Tween 60 was used instead of 400 mmol/L Tricine (pH 8.5) and 1 mass% Tween 40 in (1-9) of Example 1. A final concentration contained in the spread liquid in the case of spreading was 200 mmol/L Bicine and 0.5 mass% Tween 60. Table 6 shows results of evaluation results.

### [Comparative Example 5: Bicine and no surfactant]

Measurement was performed in the same manner as in Example 1 except that 400 mmol/L Bicine (pH 8.5) was used instead of 400 mmol/L Tricine (pH 8.5) and 1 mass% Tween 40, and a surfactant was not used in (1-9) of Example 1. A final concentration contained in the spread liquid in the case of spreading was 200 mmol/L Bicine. Table 6 shows results of evaluation results.

### [Comparative Example 6: Tris and Tween 60]

Measurement was performed in the same manner as in Example 1 except that 400 mmol/L Tris (pH 8.5) and 1 mass% Tween 60 was used instead of 400 mmol/L Tricine (pH 8.5) and 1 mass% Tween 40 in (1-9) of Example 1. A final concentration contained in the spread liquid in the case of spreading was 200 mmol/L Tris and 0.5 mass% Tween 60. Table 6 shows results of evaluation results.

Table 6 shows results of evaluating the same test for each of the examples.
ΔOD was determined according to the following criteria.
++: 0.040 or more (fully visible region)
+: 0.020 or more (visible region)
-: less than 0.020 (invisible region)

A numerical value in parentheses in the table indicates ΔOD.

**[Table 6]**

| | Surfactant | Protein | Buffer | 0 ng/mL | 0.1 ng/mL | 1.0 ng/mL |
|---|---|---|---|---|---|---|
| Example 10 | Tween 60 (0.5 mass%) | Casein (0.05 mass%) | Bicine (200 mmol/L) | (0.003) | + (0.024) | ++ (0.053) |
| Comparative Example 5 | Not contained | Casein (0.05 mass%) | Bicine (200 mmol/L) | + (0.034) | + (0.037) | ++ (0.058) |
| Comparative Example 6 | Tween 60 (0.5 mass%) | Casein (0.05 mass%) | Tris (200 mmol/L) | - (0.006) | - (0.015) | + (0.037) |

Based on these results, by using the spread liquid containing Tween 60/casein/Bicine in Examples 10, results were obtained, in which false positives due to a non-specific reaction were not generated for the negative test specimen having a concentration of 0 ng/mL, and the region was visible in the case of the positive test specimen having a low concentration of 0.1 ng/mL. Example 10 is also for comparison reasons only.

On the other hand, in the case in which Tween 60 of the compositions of Tween 60/casein/Bicine was lacking in the spread liquid in Comparative Example 5, false positives due to a non-specific reaction were generated for the negative test specimen having a concentration of 0 ng/mL in the case in which silver was amplified. Furthermore, in the case in which Bicine was changed to a Tris buffer which was not a Good's buffer in Comparative Example 6, false positives due to a non-specific reaction were not generated for the negative test specimen having a concentration of 0 ng/mL in the case in which silver was amplified, but the region was invisible in the case of the positive test specimen having a low concentration of 0.1 ng/mL. In other words, in the case in which the spread liquid contained all the compositions of Tween 60/casein/Bicine, generation of false positives was inhibited for the negative test specimen having a concentration of 0 ng/mL, a small amount of the test sample having a concentration of 0.1 ng/mL could be detected, and thereby the effect in which a dynamic range was widened during the silver amplification was confirmed. This effect is an effect that cannot be easily analogized.

### Explanation of References

1: inspection strip
2: porous carrier
3: label-holding pad (glass fiber pad)
4: liquid-sending pad
6: absorption pad
7: back pressure-sensitive adhesion sheet
9: housing case
10: upper case
12: first protrusive deforming portion
14: second protrusive deforming portion
16: hole for dropwise addition of specimen liquid
18: observation window
20: lower case
21: porous carrier accommodation portion
22: absorption pad accommodation portion
24: second pot accommodation portion
30: middle member
32: first pot accommodation portion
34: tearing portion
35: flow path-forming portion
36: rear surface of flow path-forming portion 35
40: first pot for first amplification liquid
41: first amplification liquid
42: container
43: sheet member
45: second pot for second amplification liquid
46: second amplification liquid
47: container
48: sheet member
100: chromatographic kit
L₁: inspection region
L₂: confirmation region
L₃: amplification label region
D: gap (clearance)

## Claims

1. A chromatographic kit comprising:
a label substance modified with a first binding substance for a test substance;
a nonionic surfactant;
a Good's buffer, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES;
casein;
a porous carrier including a second binding substance for the test substance or a binding substance for the first binding substance;
a compound containing silver; and
a reducing agent reducing silver ions.

2. The chromatographic kit according to claim 1, wherein the nonionic surfactant is a polysorbate surfactant.

3. The chromatographic kit according to claim 1 or 2, wherein the label substance is a metal particle.

4. The chromatographic kit according to any one of claims 1 to 3,
wherein the porous carrier includes
a label substance-holding region having the label substance modified with the first binding substance for the test substance,
a label substance-trapping region having the second binding substance for the test substance or the binding substance for the first binding substance, and
a coloring region having a coloring reagent for detecting the reducing agent reducing silver ions.

5. The chromatographic kit according to any one of claims 1 to 4, wherein the porous carrier is a nitrocellulose carrier.

6. The chromatographic kit according to any one of claims 1 to 5, wherein the first binding substance and the second binding substance are antibodies.

7. A chromatographic method comprising:
a spreading step of spreading a spread liquid onto a porous carrier including a label substance-trapping region having a second binding substance for the test substance or a binding substance for the first binding substance, the spread liquid containing a specimen containing a test substance, a label substance modified with a first binding substance for the test substance, a nonionic surfactant, a Good's buffer, and casein, wherein the Good's buffer is N-[tris(hydroxymethyl)methyl]glycine also known as Tricine, 2-hydroxy-3-[4-(2-hydroxyethyl)-1-piperazinyl]propanesulfonic acid monohydrate also known as HEPPSO, N-tris(hydroxymethyl)methyl-3-aminopropanesulfonic acid also known as TAPS, or N-cyclohexyl-2-aminoethanesulfonic acid also known as CHES;
a step of trapping a complex body of the test substance and the label substance modified with the first binding substance for the test substance in the label substance-trapping region; and
a step of amplifying a signal of the label substance of the trapped complex body using a compound containing silver and a reducing agent reducing silver ions.

8. The chromatographic method according to claim 7, wherein a final concentration of the nonionic surfactant in the spread liquid is 0.05 to 2.5 mass%.

9. The chromatographic method according to claim 7 or 8, wherein the nonionic surfactant is a polysorbate surfactant.

10. The chromatographic method according to any one of claims 7 to 9, wherein the label substance is a metal particle.

11. The chromatographic method according to any one of claims 7 to 10, the method further comprising detecting a label substance having an average particle size of equal to or more than 1 µm and equal to or less than 20 µm in a case of detection.

12. The chromatographic method according to any one of claims 7 to 11, wherein the porous carrier is a nitrocellulose carrier.

13. The chromatographic method according to any one of claims 7 to 12, wherein the specimen containing the test substance is nasal discharge, a nasal swab, a pharyngeal swab, a nasal aspirate, or urine.

## Patentansprüche

1. Chromatographie-Kit, umfassend:
eine Markierungssubstanz, die mit einer ersten Bindungssubstanz für eine Testsubstanz modifiziert ist;
ein nicht ionisches Tensid;
einen Good-Puffer, wobei der Good-Puffer N-[tris(hydroxymethyl)methyl]glycin, das auch als Tricin bekannt ist, 2-Hydroxy-3-[4-(2-hydroxyethyl)-1-piperazinyl]propansulfonsäure-Monohydrat, das auch als HEPPSO bekannt ist, N-tris(hydroxymethyl)methyl-3-Aminopropansulfonsäure, die auch als TAPS bekannt ist, oder N-cyclohexyl-2-Aminoethansulfonsäure, auch als CHES bekannt ist, ist;
Kasein;
einen porösen Träger, der eine zweite Bindungssubstanz für die Testsubstanz oder eine Bindungssubstanz für die erste Bindungssubstanz enthält;
eine Verbindung, die Silber enthält; und
ein Reduktionsmittel, das Silberionen reduziert.

2. Chromatographie-Kit nach Anspruch 1, wobei das nicht ionische Tensid ein Polysorbat-Tensid ist.

3. Chromatographie-Kit nach Anspruch 1 oder 2, wobei die Markierungssubstanz ein Metallpartikel ist.

4. Chromatographie-Kit nach einem der Ansprüche 1 bis 3,
wobei der poröse Träger enthält:
einen Markierungssubstanz-Haltebereich, der die mit der ersten Bindungssubstanz für die Testsubstanz modifizierte Markierungssubstanz aufweist,
einen Markierungssubstanz-Einfangbereich, der die zweite Bindungssubstanz für die Testsubstanz oder die Bindungssubstanz für die erste Bindungssubstanz aufweist, und einen Färbebereich, der eine Färbereagenz zum Detektieren des Reduktionsmittels, das Silberionen reduziert.

5. Chromatographie-Kit nach einem der Ansprüche 1 bis 4, wobei der poröse Träger ein Nitrocellulose-Träger ist.

6. Chromatographie-Kit nach einem der Ansprüche 1 bis 5, wobei die erste Bindungssubstanz und die zweite Bindungssubstanz Antikörper sind.

7. Chromatographieverfahren, umfassend:
einen Verteilungsschritt des Verteilens einer Verteilungsflüssigkeit auf einen porösen Träger, der einen Markierungssubstanz-Einfangbereich mit einer zweiten Bindungssubstanz für die Testsubstanz oder einer Bindungssubstanz für die erste Bindungssubstanz enthält, wobei die Verteilungsflüssigkeit eine Probe, die eine Testsubstanz enthält, eine Markierungssubstanz, die mit einer ersten Bindungssubstanz für die Testsubstanz modifiziert ist, ein nicht ionisches Tensid, einen Good-Puffer und Kasein enthält, wobei der Good-Puffer N-[tris(hydroxymethyl)methyl]glycin, das auch als Tricin bekannt ist, 2-Hydroxy-3-[4-(2-hydroxyethyl)-1-piperazinyl]propansulfonsäure-Monohydrat, das auch als HEPPSO bekannt ist, N-tris(hydroxymethyl)methyl-3-Aminopropansulfonsäure, die auch als TAPS bekannt ist, oder N-cyclohexyl-2-Aminoethansulfonsäure, die auch als CHES bekannt ist, ist.
einen Schritt des Einfangens eines komplexen Körpers der Testsubstanz und der mit der ersten Bindungssubstanz für die Testsubstanz modifizierten Markersubstanz in dem Markierungssubstanz-Einfangbereich; und
einen Schritt des Verstärkens eines Signals der Markierungssubstanz des eingefangenen komplexen Körpers unter Verwendung einer Verbindung, die Silber enthält, und eines Reduktionsmittels, das Silberionen reduziert.

8. Chromatographieverfahren nach Anspruch 7, wobei eine Endkonzentration des nicht ionischen Tensids in der Verteilungsflüssigkeit 0,05 bis 2,5 Massen-% beträgt.

9. Chromatographieverfahren nach Anspruch 7 oder 8, wobei das nicht ionische Tensid ein Polysorbat-Tensid ist.

10. Chromatographieverfahren nach einem der Ansprüche 7 bis 9, wobei die Markierungssubstanz ein Metallpartikel ist.

11. Chromatographieverfahren nach einem der Ansprüche 7 bis 10, wobei das Verfahren ferner Detektieren einer Markierungssubstanz mit einer durchschnittlichen Partikelgröße von gleich oder größer als 1 µm und gleich oder kleiner als 20 µm in einem Fall von Detektion umfasst.

12. Chromatographieverfahren nach einem der Ansprüche 7 bis 11, wobei der poröse Träger ein Nitrocellulose-Träger ist.

13. Chromatographieverfahren nach einem der Ansprüche 7 bis 12, wobei die Probe, die die Testsubstanz enthält, Nasensekret, ein Nasenabstrich, ein Rachenabstrich, ein Nasenaspirat oder Urin ist.

## Revendications

1. Kit chromatographique comprenant :
une substance d'étiquetage modifiée avec une première substance de liaison pour une substance d'essai ;
un tensioactif non ionique ;
un tampon de Good, dans lequel le tampon de Good est N-[tris(hydroxyméthyl)méthyl]glycine également connu sous le nom de Tricine, acide 2-hydroxy-3-[4-(2-hydroxyéthyl)-1-pipérazinyl]propanesulfonique monohydraté également connu sous le nom de HEPPSO, acide N-tris(hydroxyméthyl)méthyl-3-aminopropanesulfonique également connu sous le nom de TAPS, ou acide N-cyclohexyl-2-aminoéthanesulfonique également connu sous le nom de CHES ;
caséine ;
un support poreux incluant une deuxième substance de liaison pour la substance d'essai ou une substance de liaison pour la première substance de liaison ;
un composé contenant de l'argent ; et
un agent réducteur réduisant des ions argent.

2. Kit chromatographique selon la revendication 1, dans lequel le tensioactif non ionique est un tensioactif polysorbate.

3. Kit chromatographique selon la revendication 1 ou la revendication 2, dans lequel la substance d'étiquetage est une particule métallique.

4. Kit chromatographique selon l'une quelconque des revendications 1 à 3,
dans lequel le support poreux inclut
une région de rétention de substance d'étiquetage ayant la substance d'étiquetage modifiée avec la première substance de liaison pour la substance d'essai,
une région de piégeage de substance d'étiquetage ayant la deuxième substance de liaison pour la substance d'essai ou la substance de liaison pour la première substance de liaison, et
une région de coloration ayant un réactif de coloration pour détecter l'agent réducteur réduisant des ions argent.

5. Kit chromatographique selon l'une quelconque des revendications 1 à 4, dans lequel le support poreux est un support en nitrocellulose.

6. Kit chromatographique selon l'une quelconque des revendications 1 à 5, dans lequel la première substance de liaison et la deuxième substance de liaison sont des anticorps.

7. Procédé chromatographique comprenant :
une étape de répartition consistant à répartir un liquide de répartition sur un support poreux incluant une région de piégeage de substance d'étiquetage ayant une deuxième substance de liaison pour la substance d'essai ou une substance de liaison pour la première substance de liaison, le liquide de répartition contenant un échantillon contenant une substance d'essai, une substance d'étiquetage modifiée avec une première substance de liaison pour la substance d'essai, un tensioactif non ionique, un tampon de Good, et de la caséine, dans lequel le tampon de Good est N-[tris(hydroxyméthyl)méthyl]glycine également connue sous le nom de Tricine, acide 2-hydroxy-3-[4-(2-hydroxyéthyl)-1-pipérazinyl]propanesulfonique monohydraté également connu sous le nom de HEPPSO, acide N-tris(hydroxyméthyl)méthyl-3-aminopropanesulfonique également connu sous le nom de TAPS, ou acide N-cyclohexyl-2-aminoéthanesulfonique également connu sous le nom de CHES ;
un étape de piégeage d'un corps complexe de la substance d'essai et de la substance d'étiquetage modifiée avec la première substance de liaison pour la substance d'essai dans la région de piégeage de substance d'étiquetage ; et
une étape d'amplification d'un signal de la substance d'étiquetage du corps complexe piégé en utilisant un composé contenant de l'argent et un agent réducteur réduisant des ions argent.

8. Procédé chromatographique selon la revendication 7, dans lequel une concentration finale du tensioactif non ionique dans le liquide de répartition est de 0,05 à 2,5 % en masse.

9. Procédé chromatographique selon la revendication 7 ou la revendication 8, dans lequel le tensioactif non ionique est un tensioactif polysorbate.

10. Procédé chromatographique selon l'une quelconque des revendications 7 à 9, dans lequel la substance d'étiquetage est une particule métallique.

11. Procédé chromatographique selon l'une quelconque des revendications 7 à 10, le procédé comprenant en outre détection d'une substance d'étiquetage ayant une taille moyenne de particule égale ou supérieure à 1 µm et égale ou inférieure à 20 µm dans une cas d'une détection.

12. Procédé chromatographique selon l'une quelconque des revendications 7 à 11, dans lequel le support poreux est un support en nitrocellulose.

13. Procédé chromatographique selon l'une quelconque des revendications 7 à 12, dans lequel l'échantillon contenant la substance d'essai est un écoulement nasal, un écouvillon nasal, un écouvillon pharyngé, un aspirat nasal ou d'urine.
